(19) Europäisches Patentamt
European Patent Office
Office européen des brevets

(11) Veröffentlichungsnummer: **0 143 305**
**A1**

(12) **EUROPÄISCHE PATENTANMELDUNG**

(21) Anmeldenummer: **84112437.3**

(22) Anmeldetag: **16.10.84**

(51) Int. Cl.⁴: **A 61 K 47/00**, A 61 K 9/00, A 61 K 31/44, A 61 K 31/415, A 61 K 9/10

(30) Priorität: **28.10.83 DE 3339236**

(43) Veröffentlichungstag der Anmeldung: **05.06.85 Patentblatt 85/23**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Serno, Peter, Dr., An der Ruthen 3, D 5000 Koln 80 (DE)**

(54) **Arzneimittelzubereitung.**

(57) Arzneimittelzubereitung zur intravenösen Injektion enthaltend Arzneiwirkstoff, Fettphase, Emulgator und Wasser, wobei die Fettphase in Mengen bis zu 30 Gew.-%, der Emulgator in Mengen von 0,1 bis 10 Gew.-% und Wasser ad 100% vorhanden ist und die Fettphase bis zu 50 Gew.-% eines schwerlöslichen Arzneiwirkstoffs enthält und aus

a) Estern vorwiegend mittelkettiger Fettsäuren, oder

b) Mischungen von Estern vorwiegend mittelkettiger Fettsäuren mit pflanzlichen oder tierischen Ölen mit einem Anteil der Ester vorwiegend mittelkettiger Fettsäuren von mindestens 10 Gew.-%, oder

c) Estern vorwiegend mittelkettiger Fettsäuren, pflanzlichen oder tierischen Ölen oder deren Mischungen in Kombination mit 0,3 bis 200 Gew.-% Benzylalkohol bezogen auf den Fett- bzw. Ölgehalt

besteht, sowie Verfahren zu ihrer Herstellung.

EP 0 143 305 A1

0143305

BAYER AKTIENGESELLSCHAFT
5090 Leverkusen, Bayerwerk
Konzernverwaltung RP
Patentabteilung
E/by-c

## Arzneimittelzubereitung

Die Erfindung betrifft eine Arzneimittelzubereitung zur intravenösen Injektion, sowie ein Verfahren zu ihrer Herstellung.

Arzneiwirkstoffe können bekanntlich nur in gelöster Form intravenös appliziert werden. Wirkstoffe, die nur eine geringe oder keine Wasserlöslichkeit besitzen, müssen durch lösungsvermittelnde Maßnahmen in Lösung gebracht werden.

Die bisher bekannt gewordenen Maßnahmen, wie der Zusatz organischer Lösungsmittel, erwiesen sich als nicht befriedigend, da als Folge dieser Zusätze ungünstige physiologische Nebenwirkungen wie Injektionsschmerz, Thrombophlebitis, histaminoide Reaktionen, Bronchospasmen oder cardiovaskulärer Kollaps auftreten können.

Gemäß der DE-AS 1 792 410 können diese Nachteile vermieden werden, wenn die schwerlöslichen Arzneistoffe

in Form einer Fettemulsion appliziert werden, welche aus einem pflanzlichen oder tierischen Öl, insbesondere Sojabohnenöl, bestehen, das unter Zusatz von Emulgatoren und Isotonisierungsmittel in Wasser dispergiert ist.

Dieses Verfahren setzt allerdings voraus, daß sich die schwerlöslichen Arzneistoffe in hinreichender Menge in pflanzlichen oder tierischen Ölen, insbesondere Sojabohnenöl, lösen, was in den meisten Fällen jedoch nicht gewährleistet ist.

Das geringe Lösungsvermögen für schwer wasserlösliche Arzneiwirkstoffe führt zur Anwendung an sich unerwünscht hoher Zusatzmengen. Bedingt durch diese hohen Zusatzmengen wird dem Körper unvermeidbar, bei vorgegebener Wirkstoffmenge, eine hohe Flüssigkeitsmenge zugeführt. Bedingt durch den mit der Applikation verbundenen hohen Fettgehalt erfolgt ebenfalls eine unerwünscht hohe calorische Energiezufuhr.

Es wurde nun gefunden, daß eine erhebliche Steigerung der Löslichkeit schwer wasserlöslicher Arzneiwirkstoffe in Fettemulsionen durch folgende Maßnahmen erreicht werden kann:

(1) Verwendung von Estern vorwiegend mittelkettiger Fettsäuren als Bestandteile der Fettphase der Emulsion, oder

(2) Zusatz von Benzylalkohol zur Fettemulsion oder

(3) Kombinierte Verwendung von Estern vorwiegend mittelkettiger Fettsäuren und Benzylalkohol.

Die Erfindung betrifft somit eine Arzneimittelzubereitung zur intravenösen Injektion enthaltend Arzneiwirkstoff, Fettphase, Emulgator und Wasser, wobei die Fettphase in Mengen bis zu 30 Gew.-%, bevorzugt 5 bis 20 Gew.-%, der Emulgator in Mengen von 0,1 bis 10 Gew.-% und Wasser ad 100 % vorhanden ist und die Fettphase bis zu 50 Gew.-% eines schwerlöslichen Arzneiwirkstoffs enthält und aus

a) Estern vorwiegend mittelkettiger Fettsäuren, oder

b) Mischungen von Estern vorwiegend mittelkettiger Fettsäuren mit pflanzlichen oder tierischen Ölen mit einem Anteil der Ester vorwiegend mittelkettiger Fettsäuren von mindestens 10 Gew.-%, bevorzugt mindestens 30 Gew.-%, oder

c) Estern vorwiegend mittelkettiger Fettsäuren, pflanzlichen oder tierischen Ölen oder deren Mischungen in Kombination mit 0,3 bis 200 Gew.-% Benzylalkohol bezogen auf den Fett- bzw. Ölgehalt

besteht.

Die Wasserphase kann neben Wasser für Injektionszwecke bis zu 10 Gew.-% übliche Isotonisierungsmittel wie Glycerin oder Xylit enthalten.

Als Emulgatoren kommen die physiologisch verträglichen Emulgatoren in Betracht, wie Phospholipide, Polyoxyethylen-polyoxypropylen-Mischpolymerisate, Polyethylenglykolsorbitanfettsäureester, Polyethylenglykolfettsäureester, Cholesterin, Cholesterinester sowie Natriumsalze von Fettsäuren.

Als Ester vorwiegend mittelkettiger Fettsäuren seien genannt:
Mono-, Di- oder Polyester einwertiger oder mehrwertiger, bevorzugt 1 bis 3-wertiger, gesättigter $C_1$-$C_{18}$, bevorzugt $C_2$-$C_6$, aliphatischer Alkohole wie z.B. Methanol,

Ethanol, Ethandiol, Propanol, Propandiol, Propantriol, Butanol, Butan-(di-, tri-, tetra-)ol, Pentanol, Pentan-(di-, tri-, penta-)ol, Hexanol, Hexan-(di-, tri-, tetra-, penta-, hexa-)ol, Oktanol, Dekanol, Dodekanol, Tetradekanol, Hexadekanol, und Oktadekanol.

Bevorzugt seien Glycerin und 1,2-Propylenglykol genannt.

Als Säurekomponente der Ester dienen (bezogen auf die Gesamtanzahl der Säuren) 55 bis 100 Gew.-% ein- oder zweiwertige, gesättigte oder ungesättigter $C_6$-$C_{12}$, bevorzugt $C_8$-$C_{10}$ Fettsäuren (= mittelkettige Fettsäuren) und 0 bis 45 Gew.-% ein-, zwei- oder mehrwertiger, gesättigte oder ungesättigte $C_1$-$C_5$ oder $C_{13}$-$C_{22}$ Carbonsäuren.

Derartige mittelkettige Fettsäuren sind beispielsweise:

Hexansäure, Hexensäure, Hexadiensäure, Octansäure, Octensäure, Octa(di-, tri-)ensäure, Dekansäure, Dekensäure, Deka(di-, tri-, tetr-)ensäure, Dodekansäure, Dodekensäure, Dodeka(di-, tri-, tetr-, pent-)ensäure sowie alle analogen Disäuren.

Derartige Carbonsäuren sind beispielsweise:

Ameisensäure, Essigsäure, Propionsäure, Buttersäure, Valeriansäure, Myristinsäure, Palmitinsäure, Stearinsäure, Arachidinsäure, Behensäure, Butensäure, Hexadecensäure, Ölsäure, Docosensäure, Linolsäure, Linolensäure, Arachidonsäure, Malonsäure, Bernsteinsäure, Glutarsäure, Suberinsäure, Butendisäure, Glykolsäure, Milchsäure, Ricinolsäure, Äpfelsäure, Weinsäure, Citronensäure.

Als pflanzliche oder tierische Öle seien beispielsweise Sojabohnenöl, Saffloröl und/oder Baumwollsaatöl genannt.

Als schwer wasserlösliche Arzneiwirkstoffe seien beispielsweise aufgeführt:

Corticoide wie Cortisonacetat, Hydrocortison, Dexamethason, Triamcinolonacetonid

Benzodiazepine wie Diazepam, Flunitrazepam,

Antiepileptika wie Diphenylhydantoin, Clonazepam,

Chemotherapeutika wie Nitrofurantoin, Sulfamethoxazol, Trimethoprim

Antimykotika wie Griseofulvin, Amphotericin B

Herzglykoside wie Digoxin, Deslanosid

Mutterkornalkaloide wie Dihydroergotaminmesilat, Ergotamintartrat

Zytostatika wie Melphalan

Barbiturate wie Pentobarbital-Natrium

sowie fettlösliche Vitamine wie Vitamin A, $B_2$, $B_6$, $B_{12}$, E oder $K_1$.

Von ganz besonderer Bedeutung sind die Dihydropyridinverbindungen, insbesondere die mit folgender allgemeiner Formel

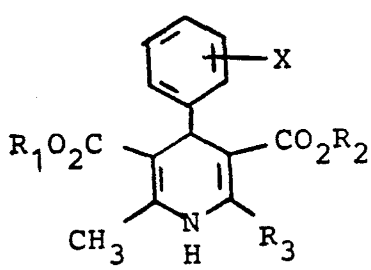

(I)

in der

$R_1$ $C_1$-$C_4$-Alkyl, gegebenenfalls substituiert durch $C_1$-$C_3$-Alkoxy,

$R_2$ $C_1$-$C_{10}$-Alkyl, gegebenenfalls substituiert durch $C_1$-$C_3$-Alkoxy, Trifluormethyl, N-Methyl-N-benzylamino,

$R_3$ $C_1$-$C_4$-Alkyl, Cyano, Hydroxymethyl und

X 2- bzw. 3-Nitro, 2,3-Dichlor, 2, 3 Ringglied bestehend aus =N-O-N=,

bedeuten.

Ganz besonders in Betracht kommen die Verbindungen der folgenden Tabelle:

Tabelle

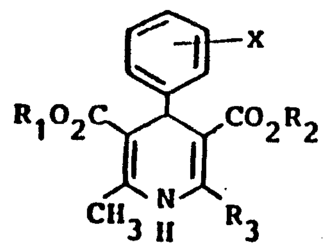

| Nr. | X | $R^1$ | $R^2$ | $R^3$ | Generic |
|---|---|---|---|---|---|
| 1 | $2-NO_2$ | $CH_3$ | $CH_3$ | $CH_3$ | Nifedipin |
| 2 | $3-NO_2$ | $nPrOCH_2CH_2$ | $nPrOCH_2CH_2$ | $CH_3$ | Niludipin |
| 3 | $3-NO_2$ | $C_2H_5$ | $CH_3$ | $CH_3$ | Nitrendipin |
| 4 | $2-NO_2$ | $CH_3$ | $(CH_3)_2CHCH_2$ | $CH_3$ | Nisoldipin |
| 5 | $3-NO_2$ | $CH(CH_3)_2$ | $(CH_2)_2-O-CH_3$ | $CH_3$ | Nimodipin |
| 6 | $3-NO_2$ | $C_2H_5$ | $C_{10}H_{21}(n)$ | $CH_3$ | |
| 7 | 2-Cl | $CH_3$ | $CH_2-CF_3$ | $CH_3$ | |
| 8 | 2-Cl | $C_2H_5$ | $CH_2-CF_3$ | $CH_3$ | |
| 9 | $3-NO_2$ | $CH(CH_3)_2$ | $n-PrO-CH_2CH_2$ | $CH_3$ | |
| 10 | $3-NO_2$ | $CH_3$ | $C_6H_5CH_2N(CH_3)CH_2CH_2$ | $CH_3$ | Nicardipin |
| 11 | $2,3-Cl_2$ | $C_2H_5$ | $CH_3$ | $CH_3$ | Felodipin |
| 12 | 2,3=N-O-N= | $C_2H_5$ | $C_2H_5$ | $CH_3$ | |
| 13 | 2,3=N-O-N= | $CH_3$ | $CH(CH_3)_2$ | $CH_3$ | |
| 14 | $3-NO_2$ | $C_2H_5$ | $C_2H_5$ | $CH_2OH$ | |
| 15 | $3-NO_2$ | $CH_3$ | $CH_3$ | CN | |

n-Pr = n-Propyl

Insbesondere seien genannt: Nifedipin, Nimodipin, Nitrendipin und Nisoldipin.

Ebenfalls von Bedeutung sind Imidazol-Derivate der Formel

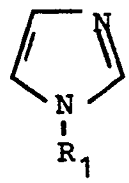

wobei

$R_1$ gegebenenfalls Chlor-, Hydroxy- oder Arylalkoxy- substituierte Arylalkylketten bedeutet.

Ganz besonders in Betracht kommen Verbindungen mit folgender Substituierung:

$R_1$ = 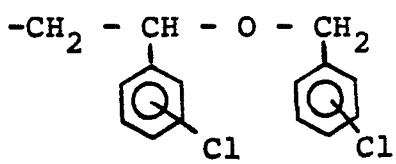 wie Miconazole, Econazole, Isoconazole

oder 2 x Cl  oder 2 x Cl

$R_1$ = 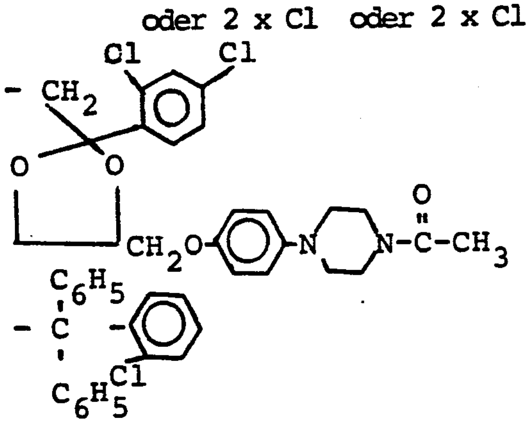 Ketoconazole

$R_1$ = Clotrimazole

$R_1$ = 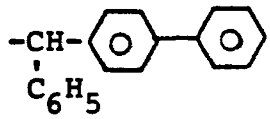 Bifonazole

Außerdem Muzolimine und Propanidid.

Die Herstellung der erfindungsgemäßen Arzneimittelzubereitung erfolgt zweckmäßigerweise in einem Zweistufenverfahren, wobei zunächst eine Voremulgierung erfolgt, an die ein Homogenierungsverfahren sich anschließt.

Die Voremulsion wird hergestellt, indem man die lipophilen Bestandteile wie die Ester vorwiegend mittelkettiger Fettsäuren, die pflanzlichen oder tierischen Öle, den Arzneiwirkstoff, sowie gegebenenfalls Benzylalkohol in den entsprechenden Mengenverhältnissen bei Temperaturen bis zu 75°C bis zum Entstehen einer homogenen Lösung oder Dispersion intensiv vermischt, und anschließend diese Mischung mit Wasser, welches gegebenenfalls das Isotonierungsmittel enthält, intensiv vermischt und

gegebenenfalls in einer Wirbelkammer vorzerkleinert, wobei der Emulgator entweder der liphophilen Phase oder der Wasserphase oder in entsprechenden Teilmengen beiden Phasen zugeführt wird. Der pH der Voremulsion wird auf einen physiologischen und zur Emulgierung optimalen Wert, beispielsweise 7,6 eingestellt.

Diese Voremulsion wird anschließend homogenisiert, bis eine Emulsion mit einem durchschnittlichen Durchmesser der Fetttröpfchen von weniger als 2 µm, insbesondere weniger als 1 µm entsteht.

Die Homogenisierung kann beispielsweise durch Hochdruckhomogenisatoren bei Drucken bis zu 1000 bar, vorzugsweise 400 bar oder durch Ultraschallgerät erfolgen.

Wie bereits geschildert, besteht der Vorteil der erfindungsgemäßen Arzneimittelzubereitung gegenüber herkömmlichen intravenösen Formulierungen schwer wasserlöslicher Arzneistoffe unter anderem darin, daß weniger Fettmengen zur Lösung des schwer wasserlöslichen Arzneiwirkstoffes erforderlich sind. Bedingt durch die geringeren Fettmengen kann das Applikationsvolumen soweit erniedrigt werden, daß eine Arzneimitteltherapie an Menschen ermöglicht wird. Zahlreiche Nachteile einer zu hohen intravenösen Fettzufuhr, wie Hyperalimentation, Anämie, Beeinflussung des Blutgerinnungssystems und Beeinträchtigung von Leberfunktionen können vermieden werden.

Wie aus der nachstehenden Tabelle 1 hervorgeht, weisen die beispielhaft untersuchten Arzneiwirkstoffe in den Emulsionen einiger beispielhaft ausgesuchter Emulsionen

von Estergemischen verschiedener mittelkettiger Fettsäuren um das doppelte bis über vierzigfach höhere Löslichkeiten auf als in Sojabohnenölemulsionen.

Tabelle 1

Vergleich der Löslichkeiten schwer wasserlöslicher Arzneistoffe in Emulsionen von Sojaöl und verschiedenen Estern vorwiegend mittelkettiger Fettsäuren
(Fettgehalt der Emulsionen 20 Gew.-%, Werte bei Raumtemperatur)

| | Arzneistofflöslichkeit in der Emulsion % (G/G) | | | | | |
|---|---|---|---|---|---|---|
| Fettphase der Emulsion | Nifedipin | Nimodipin | Nitrendipin | Nisoldipin | Muzolimine | Propanidid |
| Estergemisch A vorw. mittelkettiger Fettsäuren | 0,08 % | 0,16 % | 0,08 % | 0,14 % | 0,06 % | unbegrenzt mischbar |
| Estergemisch B vorw. mittelkettiger Fettsäuren | 0,10 % | 0,26 % | 0,12 % | 0,18 % | 0,06 % | unbegrenzt mischbar |
| Estergemisch C vorw. mittelkettiger Fettsäuren | 0,10 % | 0,50 % | 0,14 % | 0,20 % | 0,32 % | unbegrenzt mischbar |
| Estergemisch D | 0,04 % | 0,08 % | 0,05 % | 0,08 % | 0,02 % | |
| Sojabohnenöl | 0,02 % | 0,04 % | 0,03 % | 0,04 % | 0,01 % | 2,80 % |

Estergemisch A: Glycerintriester der Caprylsäure (70 %) und Caprinsäure (30 %)

Estergemisch B: Propylenglykoldiester der Caprylsäure (75 %) und Caprinsäure (25 %)

Estergemisch C: 75 % Glycerintriester der Caprylsäure (70 %) und Caprinsäure (30 %) 12,5 % Glycerindiester der Caprylsäure (60 %) und Caprinsäure (40 %) 12,5 % Glycerinmonoester der Caprylsäure (60 %) und Caprinsäure (40 %)

Estergemisch D: 50 % Sojabohnenöl und 50 % Glycerintriester der Caprylsäure (70 %) und Caprinsäure (30 %).

Die durch die weitere Verwendung von Benzylalkohol erzielbare Verbesserung der Arzneiwirkstofflöslichkeit in Emulsionen von Sojabohnenöl und/oder Estern vorwiegend mittelkettiger Fettsäuren geht aus Tabelle 2 hervor. Wie den angeführten Daten zu entnehmen ist, führt bereits eine Konzentration von 5 Gew.-% Benzylalkohol in parenteralen Fettemulsionen zu einer Erhöhung der Arzneistofflöslichkeit um den Faktor 2,8 bis 4,5.

Tabelle 2

Steigerung der Löslichkeit eines schwer wasserlöslichen Arzneistoffes in parenteralen Fettemulsionen durch Zusatz von Benzylalkohol (Werte bei Raumtemperatur, Fettgehalt der Emulsion 20 %)

| Benzylalkoholgehalt der Emulsion (% G/G) | Löslichkeit von Nimodipin in Emulsion aus Estern vorwiegend mittelkettiger Fettsäuren* | Löslichkeit von Nimodipin in Sojabohnenölemulsion |
|---|---|---|
| 0 % | 0,16 % | 0,04 % |
| 2 % | 0,22 % | 0,08 % |
| 4 % | 0,36 % | 0,16 % |
| 5 % | 0,46 % | 0,18 % |

* verwendet wurden Triglyceride mittelkettiger Fettsäuren mit einem Anteil von 70 % Caprylsäure und 30 % Caprinsäure.

Weitere Vorteile einer Verwendung von Estern vorwiegend mittelkettiger Fettsäuren und/oder Benzylalkohol bestehen darin, daß nach Homogenisierung kleinere Tröpfchendurchmesser und somit eine verbesserte Emulsionsstabilität erzielt werden. Bei Verwendung von Estern vorwiegend gesättigter mittelkettiger Fettsäuren tritt außerdem eine Verbesserung der Stabilität gegenüber Fettoxidation ein.

Die Herstellung der Arneimittelzubereitungen sei anhand folgender Beispiele erläutert:

Beispiel 1

24,000 g gereinigte Eigelbphosphatide werden in einer Mischung aus 50,000 g gereinigtem Sojabohnenöl für Injektionszwecke und 50,000 g Glycerintriester mittelkettiger Fettsäuren der Fettsäurezusammensetzung aus 60 Gew.-% Caprylsäure, 40 Gew.-% Caprinsäure bei 60 bis 70°C homogen dispergiert. Dieser Mischung wird unter Lichtschutz 0,100 g Nifedipin zugesetzt. Nach Einrühren von 7,500 g Glycerin und 292,500 g Wasser für Injektionszwecke entsteht eine Voremulsion, welche nach Einstellen des pH auf 8,2 unter Verwendung eines handelsüblichen Hochdruckhomogenisators bei 400 bar in 5 Durchgängen homogenisiert wird. Die entstehende Feinstemulsion wird bei 20°C auf ein Volumen von 1 Liter mit Wasserphase bestehend aus 975,000 g Wasser für Injektionszwecke und 25,000 g Glycerin aufgefüllt, filtriert und in lichtgeschützte Injektionsflaschen zu 50 ml unter Stickstoffbegasung abgefüllt.

Beispiel 2

6,000 g gereinigte Sojabohnenphosphatide werden in einer Mischung aus 50,000 g gereinigtem Sojabohnenöl für Injektionszwecke und 50,000 g Benzylalkohol bei 50°C gelöst. In dieser Lösung wird unter Lichtschutz 0,100 g Nisoldipine aufgelöst. Nach Einrühren von 150,000 g Wasser für Injektionszwecke entsteht eine Voremulsion, welche nach Einstellen des pH auf 7,6 die wie im Beispiel 1 beschrieben homogenisiert wird. Nach Auffüllen mit Wasser für Injektionszwecke auf 1 Liter bei 20°C wird in lichtgeschützte Ampullen zu 2 ml unter Lichtschutz und Stickstoffbegasung abgefüllt.

Beispiel 3

15,000 g gereinigte Sojabohnenphosphatide werden in einer Mischung aus 200 g Glycerintriestern der Caprylsäure (50 Gew.-%), Caprinsäure (34 Gew.-%) und Bernsteinsäure (16 Gew.-%) und 10,000 g Benzylalkohol bei 50°C gelöst. In dieser Lösung wird unter Lichtschutz 0,500 g Nitrendipin aufgelöst. Nach Einrühren von 12,000 g Glycerin und 588,000 g Wasser entsteht eine Voremulsion, die wie im Beispiel 1 beschreiben homogenisiert wird. Nach Auffüllen mit Wasserphase bestehend aus 980 g Wasser für Injektionszwecke und 20 g Glycerin auf 1 Liter bei 20°C wird in lichtgeschützte Ampullen zu 10 ml unter Lichtschutz und Stickstoffbegasung abgefüllt.

Beispiel 4

2,000 g Muzolimine werden in einer Mischung aus 150,000 g Glycerintriester der Caprylsäure (70 Gew.-%) und Caprinsäure (30 Gew.-%), 25,000 g Glycerindiester der Caprylsäure (60 Gew.%) und Caprinsäure (40 Gew.-%) und 25,000 g Glycerinmonoester der Caprylsäure (60 Gew.-%) und Caprinsäure (40 Gew.-%) gelöst. Diese Lösung wird in 600,00 g Wasserphase bestehend aus 16,000 g Pluronic [R] F 68 und 584,000 g Wasser für Injektionszwecke bei 50°C eingerührt. Nach Homogenisieren wie in Beispiel 1 beschrieben wird bei 20°C auf 1 Liter mit Wasser für Injektionszwecke aufgefüllt und in Ampullen zu 10 ml unter Stickstoffbegasung abgefüllt.

Beispiel 5

7,2000 g gereinigte Eigelbphosphatide werden in 60,000 g Glycerintriester der Caprylsäure (50 Gew.-%), der Caprinsäure (40 Gew.-%), der Linolsäure (5 Gew.-%) sowie Capron- und Laurinsäure (zusammen 5 Gew.-%) bei 60-70°C homogen dispergiert. Dieser Mischung wird unter Lichtschutz 0,400 g Nimodipin zugesetzt. Nach Eindispergieren von 5,500 g Glycerin und 175,500 g Wasser für Injektionszwecke in einer hochtourigen Wirbelkammer entsteht eine Voremulsion, die wie in Beispiel 1 beschrieben homogenisiert wird. Nach Auffüllen mit Wasserphase bestehend aus 975,000 g Wasser für Injektionszwecke und 25,000 g Glycerin auf 1 Liter bei 20°C wird in lichtgeschützte Injektionsflaschen zu 50 ml unter Lichtschutz und Stickstoffbegasung abgefüllt.

Beispiel 6

15,000 g gereinigte Sojabohnenphosphatide werden in einer Mischung aus 200,000 g Propylenglykoldiester der Caprylsäure (75 Gew.-%) und der Caprinsäure (25 Gew.-%) und 15,000 g Benzylalkohol bei 50°C gelöst. In dieser Lösung wird 2,000 g Ketoconazole aufgelöst. Nach Einrühren von 14,000 g Glycerin und 586,000 g Wasser für Injektionszwecke entsteht eine Voremulsion, die wie in Beispiel 1 beschrieben homogenisiert wird. Nach Auffüllen mit Wasser für Injektionszwecke auf 1 Liter bei 20°C wird in Ampullen zu 10 ml unter Stickstoffbegasung abgefüllt.

Beispiel 7

7,500 g gereinigte Sojabohnenphosphatide werden in 100,000 g Glycerintriester der Caprylsäure (60 Gew.-%) und der Caprinsäure (40 Gew.-%) bei 60-70°C gelöst. In dieser Lösung wird 50,000 g Propanidid aufgelöst. Nach Einrühren von 15,000 g Xylit und 285,000 g Wasser für Injektionszwecke entsteht eine Voremulsion, die wie in Beispiel 1 beschrieben homogenisiert wird. Nach Auffüllen mit Wasserphase bestehend aus 950,000 g Wasser und 50,000 g Xylit auf 1 Liter bei 20°C wird unter Stickstoffbegasung in Ampullen zu 10 ml abgefüllt.

Patentansprüche

1. Arzneimittelzubereitung zur intravenösen Injektion enthaltend Arzneiwirkstoff, Fettphase, Emulgator und Wasser, wobei die Fettphase in Mengen bis zu 30 Gew.-%, der Emulgator in Mengen von 0,1 bis 10 Gew.-% und Wasser ad 100 % vorhanden ist und die Fettphase bis zu 50 Gew.-% eines schwerlöslichen Arzneiwirkstoffs enthält und aus

   a) Estern vorwiegend mittelkettiger Fettsäuren, oder

   b) Mischungen von Estern vorwiegend mittelkettiger Fettsäuren mit pflanzlichen oder tierischen Ölen mit einem Anteil der Ester vorwiegend mittelkettiger Fettsäuren von mindestens 10 Gew.-%, oder

   c) Estern vorwiegend mittelkettiger Fettsäuren, pflanzlichen oder tierischen Ölen oder deren Mischungen in Kombination mit 0,3 bis 200 Gew.-% Benzylalkohol bezogen auf den Fett- bzw. Ölgehalt

   besteht.

2. Arzneimittelzubereitung gemäß Anspruch 1 enthaltend die Fettphase in Mengen von 5 bis 20 Gew.-% und einen

Anteil der Ester vorwiegend mittelkettiger Fettsäuren von mindestens 30 Gew.-%.

3. Arzneimittelzubereitung gemäß Ansprüchen 1 und 2 enthaltend als Ester vorwiegend mittelkettiger Fettsäuren Mono-, Di- oder Polyester ein- oder mehrwertiger, gesättigter, aliphatischer $C_1$-$C_{18}$ Alkohol mit 55 bis 100 Gew.-% ein- oder zweiwertiger, gesättigter oder ungesättigter $C_6$-$C_{12}$ Fettsäuren und 0 bis 45 Gew.-% ein-, zwei-, oder mehrwertiger gesättigter oder ungesättigter $C_1$-$C_5$ oder $C_{13}$-$C_{22}$ Carbonsäuren.

4. Arzneimittelzubereitung gemäß Anspruch 3 enthaltend als Ester vorwiegend mittelkettiger Fettsäuren solche mit einer 1 bis 3 wertigen, aliphatischen $C_2$-$C_6$ Alkoholkomponenten.

5. Arzneimittelzubereitung gemäß Anspruch 4 enthaltend als Ester vorwiegend mittelkettiger Fettsäuren solche mit Glycerin oder 1,2 Propylenglykol als Alkoholkomponente.

6. Arzneimittelzubereitung gemäß Ansprüchen 1 bis 5 enthaltend als Arzneiwirkstoff Corticoide, Benzodiazepine, Antiepileptika, Chemotherapeutika, Herzglykoside, Mutterkornalkaloide, Zytostatika, Barbiturate, fettlösliche Vitamine, Dihydropyridinverbindungen, Imidazolderivate, Triazolderivate, Muzolimine und/oder Propanidid.

7. Arzneimittelzubereitung gemäß Ansprüchen 1 bis 5 enthaltend als Arzneiwirkstoff eine Dihydropyridinverbindung der allgemeinen Formel

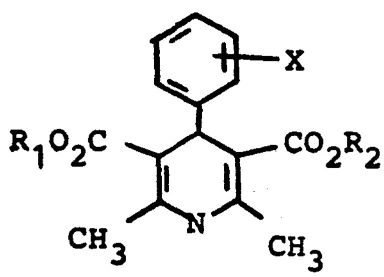

in der

$R_1$ $C_1$-$C_4$-Alkyl, gegebenenfalls substituiert durch $C_1$-$C_3$-Alkoxy,

$R_2$ $C_1$-$C_{10}$-Alkyl, gegebenenfalls substituiert durch $C_1$-$C_3$-Alkoxy, Trifluormethyl, N-Methyl-N-benzylamino,

$R_3$ $C_1$-$C_4$-Alkyl, Cyano, Hydroxymethyl und

X 2- bzw. 3-Nitro, 2,3-Dichlor, 2, 3 Ringglied bestehend aus =N-O-N=,

bedeuten.

8. Arzneimittelzubereitung gemäß Ansprüchen 1 bis 5 enthaltend als Arzneiwirkstoff ein Imidazolderivat der allgemeinen Formel

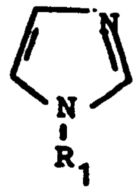

in der

$R_1$ gegebenenfalls durch Chlor, Hydroxy oder Arylalkyloxygruppen substituierte Arylalkylgruppen bedeuten.

9. Verfahren zur Herstellung der Arzneimittelzubereitungen gemäß Ansprüchen 1 bis 8, dadurch gekennzeichnet, daß man zunächst eine Voremulsion herstellt, an die sich ein Homogenisierungsverfahren anschließt, wobei zur Herstellung der Voremulsion die lipophilen Bestandteile wie die Ester vorwiegend mittelkettiger Fettsäuren, die pflanzlichen oder tierischen Öle, der Arzneiwirkstoff, sowie gegebenenfalls Benzylalkohol bei Temperaturen bis zu 75°C vermischt werden, anschließend diese

Mischung mit Wasser, welches gegebenenfalls Isotonierungsmittel enthält intensiv vermischt, wobei der Emulgator entweder der lipophilen Phase oder der Wasserphase oder in entsprechenden Teilmengen beiden Phasen zugeführt wird und anschließend, ggf. nach pH-Einstellung, die Voremulsion homogenisiert, bis eine Emulsion mit einem durchschnittlichen Fetttröpfchendurchmesser von kleiner als 1 µm entsteht.

10. Verfahren gemäß Anspruch 9, dadurch gekennzeichnet, daß die Homogenisierung durch Hochdruckhomogenisatoren bei Drücken bis zu 1000 bar oder durch Ultraschallgeräte erfolgt.

Europäisches Patentamt

**EUROPÄISCHER RECHERCHENBERICHT**

0143305

Nummer der Anmeldung

EP 84 11 2437

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| D,X | DE-A-1 792 410 (APOTEKSVARUCENTRALEN VITRUM APOTEKAREAKTIEBOLAGET) * Seite 7, Absatz 2 - Seite 8, Absatz 1; Seiten 9,10; Ansprüche * | 1-9 | A 61 K 47/00<br>A 61 K 9/00<br>A 61 K 31/44<br>A 61 K 31/415<br>A 61 K 9/10 |
| X | DE-A-1 467 929 (F. HOFFMANN-LA ROCHE & CO.) * Seiten 1,2; Seite 5, Absatz 3; Ansprüche * | 1-6 | |
| X | DE-C- 592 881 (DR. CARL WEILL) * Insgesamt * | 1-9 | |
| X | CHEMICAL ABSTRACTS, Band 91, Nr. 20, 12. November 1979, Seite 350, Nr. 163057s, Columbus, Ohio, US; & JP - A - 79 55714 (OTA PHARMACEUTICAL CO., LTD.) 04.05.1979 * Zusammenfassung * | 1-7 | **RECHERCHIERTE SACHGEBIETE (Int. Cl.4)**<br>A 61 K |
| X | DE-A-2 935 573 (LEPHARM-PHARMAZEUTISCHE ENTWICKLUNGS- UND VERTRIEBSGESELLSCHAFT GmbH) * Ansprüche; Seite 3, Absatz 3; Seite 5, Absätze 4-6 * | 1-6 | |
| | --- -/- | | |

Der vorliegende Recherchenbericht wurde für alle Patentansprüche erstellt.

| Recherchenort | Abschlußdatum der Recherche | Prüfer |
|---|---|---|
| DEN HAAG | 08-02-1985 | BERTE M.J. |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

**EUROPÄISCHER RECHERCHENBERICHT**

0143305

Nummer der Anmeldung

EP 84 11 2437

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 004 650 (BAYER) * Seite 6, Absatz 3; Seite 7, Zeilen 1-15; Ansprüche * --- | 1,6,7 | |
| A | EP-A-0 058 887 (BAYER) * Ansprüche * ----- | 1,6,8 | |
| | | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| DEN HAAG | 08-02-1985 | BERTE M.J. |

KATEGORIE DER GENANNTEN DOKUMENTEN

- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument